# EUROPEAN PATENT SPECIFICATION

(11) **EP 2 552 234 B1**
(45) Date of publication and mention of the grant of the patent: **04.12.2013**
(21) Application number: 11716063.0
(22) Date of filing: 28.03.2011
(51) Int. Cl.: A23L 1/00, A23L 1/22, A61K 8/11, A61K 9/14

(54) **SPRAY-DRIED CRYSTALLINE ACTIVE INGREDIENT**
SPRÜHGETROCKNETER KRISTALLINER WIRKSTOFF
PRINCIPE ACTIF CRISTALLIN SÉCHÉ PAR PULVÉRISATION

(30) Priority: 29.03.2010 US 318529 P
(43) Date of publication of application: 06.02.2013
(73) Proprietor: Firmenich S.A., 1211 Geneva 8 (CH)
(72) Inventor: GREGSON, Christopher M., New Jersey 08542 (US); SKIFF, Ronald H., New Jersey 08536 (US); TRIOLO, Ferdinand M., New Jersey 08536 (US)
(74) Representative: Carina, Riccardo Filippo
(86) International application number: PCT/IB2011/051294
(87) International publication number: WO 2011/121515

(56) References cited:
- WO-A1-99/52526
- WO-A1-2005/055964
- US-A1- 2009 252 789

## Description

### Technical Field

The present invention relates to a process for preparing a product comprising a crystalline active ingredient in water having excellent dissolution kinetics. The invention also relates to a product produced according to the process.

### Background and Prior Art

Many potentially useful active materials are insoluble or at best only sparingly soluble in water. This places significant limits on their usefulness or requires that they are used in combination with solvents which is almost always more expensive than water and which may have physiological incompatibilities or environmental negatives.

In addition, it is desirable for many applications that the active material dissolves rapidly in an aqueous medium. For instance, powdered beverages are diluted with water and consumed immediately, thus requiring all the ingredients to be dissolved rapidly so that the beverage has a balanced flavor. Thus, there is a need to ensure that the active ingredient dissolves sufficiently rapidly.

Various approaches have been taken to improve solubility of poorly soluble materials. A review of these approaches is given in the publication "Formulation approaches for orally administered poorly soluble drugs", Pinnamaneni S., Das N.G., Das S.K., Pharmazie, 2002, 57, 291 to 300. Such approaches include particle size reduction, modification of the crystal habit, dispersion in carriers, complexation and solubilization by surfactants.

Another review, "Improvement of solubility and dissolution rate of poorly water-soluble salicylic acid by a spray-drying technique" Kawashima Y, Saito M, Takenaka H, J. Pharm. Pharmacol. 1975, 27, 1-5 discloses spray drying a dispersion of salicylic acid in acacia solutions which is said to result in as much as a 50% improvement in the solubility of the product due not only to the concentration of acacia but also the amount of amorphous material in the spray-dried products. The dissolution rate of the spray-dried product is also reported to be almost instantaneous being about 60 times faster than that of the original powder. This is said to be due to a great improvement in the wettability of the spray-dried material.

However, for certain crystalline active ingredients, improved dissolution kinetics may not be sufficiently improved by an increase in wettability.

WO-A-2008/006712 (Unilever) discloses a process for making contra-soluble nano-dispersions of at most sparingly-soluble materials in a soluble carrier material comprising the steps of:
(i) providing a single phase mixture of : (a) a solvent or a mixture of miscible solvents, (b) at least one carrier material soluble in solvent (a), said carrier material being also contra-soluble to payload material (c) and solid at ambient temperature, (c) at least one payload material which is soluble in solvent (a), and,
(ii) drying the mixture to remove solvent (a) and thereby obtain the carrier material (b) in solid form with payload (c) dispersed therein as nanoparticles. Drying is typically performed by spray-drying.

Nevertheless, there remains a strong need to provide a simple process for improving dissolution kinetics.

The present invention seeks to address one or more of the abovementioned problems and/or to provide one or more of the abovementioned benefits.

### Summary of the Invention

Accordingly, the present invention provides a process for preparing a spray-dried product for improving the rate of dissolution of the active ingredient, the process comprising the steps of:
(i) mixing a crystalline active ingredient, which has a dissolution rate in unstirred water at 25°C of greater than 15 minutes at a concentration of 14 ppm of the active ingredient, with a starch derivative, a second carrier material, and xanthan gum to form a suspension, dispersion or solution of the active ingredient,
(ii) homogenizing the mixture at a pressure of at least 4000 psig (2.758 x 10⁷Pa), and
(iii) spray-drying the homogenized mixture,
the spray-dried active ingredient having an increased dissolution rate in water compared to the unencapsulated active ingredient.

The invention further provides spray dried particles prepared according to the above mentioned process.

### Detailed Description of the Invention

The present invention relates to a process by which the dissolution kinetics of a crystalline active ingredient can be significantly improved.

The invention is based on the combination of certain components, such as compounds or ingredients, together with defined processing conditions.

The first critical component is a starch derivative. By "starch derivative", it is meant a chemically modified starch, more preferably a hydrophobically modified starch, even more preferably an alkenyl-succinated starch.

The alkenyl-succinated starch preferably has a degree of substitution of from 0.001 to 0.9. The degree of substitution denotes the number of alkenylsuccinic functional groups per glucose units. Thus, a degree of substitution of 0.001 means that there is 1 alkenylsuccinic functional group per 1000 glucose units. The degree of substitution is more preferably from 0.005 to 0.3 and most preferably from 0.01 to 0.1, e.g. from 0.015 to 0.05.

The alkenyl-succinated starch is preferably a C3 to C14 alkenyl-succinated starch, more preferably C4 to C12, most preferably C5 to C10, e.g. C7 to C9.

Most preferably the alkenyl-succinated starch is octenyl-succinated starch. Ideally the octenyl-succinated starch has a degree of substitution no greater than 0.03, more preferably no greater than 0.02.

The dissolution kinetics of the spray dried active ingredient are found to be significantly improved when the starch derivative is present in an amount of from 7% to 25% by weight, more preferably from 7 to 20%, based on the total weight of the mixture prior to spray-drying. At a level of less than 7%, the dissolution rate of the spray-dried active ingredient is hardly improved. Whereas, in an amount greater than 25%, the rate of dissolution is severely adversely affected. Without wishing to be bound by theory, it is believed that the reduction in the rate of dissolution is due to the insolubility of an excess of the starch derivative in water.

Another critical component is the second carrier material.

The second carrier material comprises a maltodextrin, having a mean dextrose equivalence of 5 to 25, preferably 6 to 20, more preferably 10 to 19.

In a preferred embodiment, the second carrier material is present in an amount of from 40 to 80%, more preferably 60 to 80% by weight, based on the total weight of the mixture prior to spray-drying.

Xanthan gum is a polysaccharide used as a food additive and rheology modifier. It is produced by fermentation of glucose or sucrose by the *Xanthomonas campestris* bacterium.

Xanthan gum is added as a processing aid in order to maintain the insoluble crystalline solid suspended in solution prior to spray drying.

The optimum use level for maintaining an adequate suspension was found to be when the xanthan gum is present in an amount of from 0.3% to 0.6% by weight, based on the total weight of the mixture prior to spray-drying. In amounts inferior to 0.3%, it is a noticeable problem that the insoluble solids do not remain suspended in the slurry. Whereas, in amounts superior to 0.6%, the viscosity of the slurry is too high and this can adversely affect the homogenization step.

The active ingredient that is encapsulated is a compound that preferably has a dissolution rate in unstirred water at 25°C of more than 15 minutes at a concentration of 14 ppm. For the purposes of the present invention, "dissolution rate" is measured according to the method set out in the examples, described below.

The active ingredient is crystalline. By "crystalline" it is meant that the active ingredient forms a structure that exhibits long-range order in three dimensions. Crystallinity can be measured using known techniques in the art such as powder x-ray diffraction (PXRD) crystallography, solid state NMR, or thermal techniques such as differential scanning calorimetry (DSC).

The active ingredient may also be a "sparingly water-soluble organic active agent". Such an ingredient is typically a compound that has a solubility in water of less than 5% by weight, preferably less than 1% by weight, more preferably less than 0.1% by weight, even more preferably less than 0.01% by weight, in water at 20°C.

Suitable active ingredients include those for use in the foodstuff, pharmaceutical and cosmetic applications. A non-limiting list of examples includes: flavour and fragrance materials of both natural and synthetic origins, compounds and mixtures such as heliotropine, bromelia, (5RS,6RS)-2,6,10,10-tetramethyl-1-oxaspiro-[4,5]dec-6-yl acetate, acetanisole, methylsalicylique aldehyde, *para*-ethyl phenol, phenol, phenylethyl salicylate, menthol, cyclohexanecarboxamide, veratraldehyde, xylenol, dodecanoic acid, thymol, heliotropyl acetate, methyl anisate, methylnaphtylketone, myristic acid, palmitic acid, dimethylphenol, dimethyl acrylic acid, coumarine, methyl cyclopentenolone, 7-methylcoumarin, phenylacetate, acetylpyrazine, phenylacetic acid, isoeugenyl acetate, raspberry ketone, naringin, propenyl guaethol, tetramethylpyrazine, acetylmethyl carbinol, 3-hydroxy-2-ethyl-4-pyranone, malic acid, resorcinol, benzoic acid, cinnamic acid, benjoin sumatra, benjoin siam, citric acid, tartric acid, camphor, quinine chlorohydrate, ascorbic acid, borneol, glutamic acid, 5-methyl quinoxaline and malt extract; liposoluble vitamins, such as vitamin A and derivatives, vitamin D₂, vitamin D₃, natural or synthetic α-, β-, γ- or δ-tocopherol, preferably natural or synthetic α-tocopherol, and tocotrienol and tocopheryl C₁-C₂O-carboxylates; water-insoluble or sparingly water-soluble organic UV screening substances, such as, e.g., compounds from the group of the triazines, anilides, benzophenones, triazoles, cinnamides and sulfonated benzimidazoles; polyunsaturated fatty acids, such as, e.g., arachidonic acid, eicosapentaenoic acid or docosahexaenoic acid; food colorants, such as curcumine, carmine or chlorophyll; carotenoids, such as, e.g., β-carotene and lycopene; and xanthophylls, such as, e.g. lutein, astaxanthin, zeaxanthin, capsanthin, capsorubin, cryptoxanthin, citranaxanthin, canthaxanthin, bixin, β-apo-4-carotenal, β-apo-8-carotenal and β-apo-8-carotenic acid ethyl ester. The technical field of application is not critical though.

The active ingredient is preferably present in an amount of from 15 to 25% by weight, based on the total weight of the spray dried particle.

A suitable process for producing the spray-dried particles of the invention comprises a first step of forming a dispersion, suspension or solution of the starch and second carrier material. This may be performed by simple mixing of the components with water, preferably hot water at a temperature of 60°C to 80°C. Separately, a dispersion or solution is prepared of the active ingredient in a suitable solvent, such as propylene glycol. The two solutions are then mixed together.

The resulting mixture is homogenized according to defined processing parameters in order to provide a product having excellent dissolution kinetics.

Critically, the spray dried particles are prepared by a process in which a homogenization step occurs at a minimum pressure of 4000 psig, more preferably 5000 psig or more, most preferably 5500 psig or more prior to the spray drying step.

The homogenization step is preferably carried out in two or more complete passes in a standard homogenizer. A two-stage homogenizer is suitable for this purpose.

After homogenization, the mixture is subjected to a spray-drying step. The spray drying is preferably performed using nozzle atomization only. The spray pressure is preferably from about 1500 to 2500 psig. The inlet temperature is preferably from 180°C to 200°C. The outlet temperature is preferably from 70°C to 100°C.

The spray dried particles can then be collected by any standard process. Optionally and preferably they are passed through a sieve, such as a 20 Mesh screen to obtain a more homogeneous particle size.

The spray dried particles provide a significantly improved dissolution rate in a 0.05% aqueous citric acid solution of the active ingredient compared to the dissolution rate of the unencapsulated active ingredient. Further, most of the spray dried particles have an improved dissolution rate in water of the active ingredient compared to the dissolution rate of the unencapsulated active ingredient.

For instance, the dissolution rate in a 0.05% aqueous citric acid solution of the spray dried particles at 25°C is preferably less than 15 minutes at a concentration of 14 ppm of the active ingredient, more preferably less than 10 minutes, even more preferably less than 4 minutes, most preferably less than 2 minutes.

### Examples

The invention will now be described with reference to the following examples. It is to be understood that the examples are illustrative of the invention and that the scope of the invention is not limited thereto.

Samples according to the invention are denoted by a number and comparative examples by a letter.

In the examples, and more generally for the purposes of the present invention, calculation of the dissolution kinetics of the spray-dried active ingredient are performed as follows.

Detection was made using a fiber optic spectrometer, comprising a D₂Lite deuterium tungsten light source, TR 600-10 transmission probes (10mm pathlength tip) and a S2000 spectrometer (World Precision Instruments/ Ocean Optics). Data was recorded using the Ocean Optics software OOIBase32. Acquisition rate was set at one measurement per second.

The spectrum of the fully dissolved active was initially determined in the appropriate solvent (water or citric acid aq.) to find an appropriate wavelength for measurements. An amount of the spray dried powder was used for each experiment resulting in an equivalent nominal concentration of 14ppm of the active ingredient. The λmax at 324 nm was chosen as the signal wavelength. A second wavelength (400 nm) where no absorption by the active ingredient was detected was used to correct the signal for changes in baseline caused by particles, turbidity, etc.

The spray dried powders were weighed on a weighing paper and rapidly tipped into the dissolution vessel (Distek 2100B, 1 liter flask maintained at 25°C using a water-bath) containing deionized water as the solvent for one set of experiments, and 500 ppm citric acid in deionized water for a second set of experiments. The contents were stirred using paddles at 200 rpm. Experiments were run until there was no longer a noticeable increase in absorbance on a plot of absorbance versus log time. The results were normalized by dividing the absorbance at each data point with the maximum absorbance. The kinetics of dissolution were quantified by finding the time at which the normalized absorbance reached 0.95. These values are equivalent to the dissolution of 95% of the active ingredient.

The same method was used for calculating the dissolution rate of the active ingredient alone.

### Example 1

### Preparation of Spray-Dried Particles

The compositions given in table 1 were prepared as follows. The xanthan gum, maltodextrin and starch were added to the water that had been preheated to 70°C, and mixed until fully dissolved. Separately, the active ingredient was added to the propylene glycol solution and mixed for 30 minutes. The two mixtures were then combined and mixing was continued for a further 30 minutes.

The mixture was then transferred to a high pressure homogenizer and homogenization was carried out in two passes at the pressures given in the following table. The homogenized product was then spray dried under standard conditions at an inlet temperature of 171°C and an outlet temperature of 82°C. The resulting spray-dried particles were collected as a yellow powder. All amounts are parts by weight.

**Table 1**

| Sample | PG (1) | Xanthan Gum (2) | Maltodextrin 18DE (3) | Active Ingredient (4) | Starch Derivative (5) | Water | Homogenization Pressure (psig) | |
|---|---|---|---|---|---|---|---|---|
| | | | | | | | 1^{st} pass | 2^{nd} pass |
| 1 | 4.48 | 0.4 | 71.46 | 15.7 | 7.96 | 158 | 5800 | 5800 |
| 2 | 4.48 | 0.4 | 71.46 | 15.7 | 7.96 | 158 | 5800 | 5800 |
| 3 | 4.48 | 0.4 | 75.71 | 11 | 8.41 | 158 | 5800 | 5800 |
| 4 | 4.48 | 0.4 | 75.71 | 11 | 8.41 | 158 | 5800 | 5800 |
| 5 | 4.48 | 0.4 | 57.42 | 15.7 | 22 | 158 | 5800 | 5800 |
| 6 | 4.48 | 0.4 | 53.12 | 20 | 22 | 158 | 5800 | 5800 |
| 7 | 4.48 | 0.4 | 67.61 | 20 | 7.51 | 158 | 5800 | 5800 |
| 8 | 4.48 | 0.4 | 53.12 | 20 | 22 | 158 | 4000 | 4000 |
| 9 | 4.48 | 0.4 | 45.12 | 20 | 30 | 158 | 4000 | 4000 |

| | | | | | | | | |
|---|---|---|---|---|---|---|---|---|
| (1) Propylene glycol (2) ex Firmenich, ref. 946867 (3) Glucidex 19D, ex Roquette Frères (4) Poorly soluble crystalline taste-modifying active ingredient, ex Firmenich ref. 983950 (5) Capsul, ex National Starch | | | | | | | | |

The samples were then dissolved in water or an aqueous citric acid solution, as described above to provide a solution containing 14 ppm of the active ingredient.

**Table 2**

| Sample | Gram added per liter solution (to provide 14 ppm active ingredient) |
|---|---|
| 1 | 0.089 |
| 2 | 0.089 |
| 3 | 0.127 |
| 4 | 0.127 |
| 5 | 0.089 |
| 6 | 0.07 |
| 7 | 0.07 |
| 8 | 0.07 |
| 9 | 0.07 |

The rate of dissolution of the various samples was then evaluated according to the methodology described above. The results for 95% dissolution are given in the following tables:

**Table 3**

| Sample | In water (sec) | | | | |
|---|---|---|---|---|---|
| | trial 1 | trial 2 | trial 3 | trial 4 | average |
| 1 | 925 | 853 | - | - | 889 |
| 2 | 749 | 665 | - | - | 707 |
| 3 | 573 | 599 | - | - | 586 |
| 4 | 479 | 556 | - | - | 518 |
| 5 | 1162 | 1300 | - | - | 1231 |
| 6 | 721 | 818 | 802 | 622 | 741 |
| 7 | 762 | 1310 | - | - | 1036 |
| 8 | 1334 | 1184 | 1148 | 1215 | 1220 |
| 9 | 2071 | 2188 | - | - | 2130 |

**Table 4**

| Sample | In 0.05% citric acid aqueous solution (sec) | | | | |
|---|---|---|---|---|---|
| | trial 1 | trial 2 | trial 3 | trial 4 | average |
| 1 | 230 | - | - | - | 230 |
| 2 | 228 | - | - | - | 228 |
| 3 | 78 | - | - | - | 78 |
| 4 | 113 | - | - | - | 113 |
| 5 | 154 | - | - | - | 154 |
| 6 | 113 | 165 | 130 | - | 136 |
| 7 | 111 | - | - | - | 111 |
| 8 | 193 | 144 | 228 | 311 | 219 |
| 9 | 220 | 323 | - | - | 272 |

The active ingredient alone had a dissolution rate of 1106 seconds in water at 25°C and of 437 seconds in 0.05% aqueous citric acid solution. Thus, the spray dried systems comprising the active ingredient show a remarkable improvement in dissolution kinetics in all cases when the spray dried active ingredient was dissolved in a dilute aqueous citric acid solution and in most cases where the spray dried active ingredient was dissolved in water.

## Claims

1. A process for preparing a spray-dried product for improving the rate of dissolution of the active ingredient, the process comprising the steps of:
(i) mixing a crystalline active ingredient, which has a dissolution rate in unstirred water at 25°C of greater than 15 minutes at a concentration of 14ppm of the active ingredient, with a starch derivative, a second carrier material comprising a maltodextrin having a mean dextrose equivalence of 5 to 25, and xanthan gum to form a suspension, dispersion or solution of the active ingredient,
(ii) homogenizing the mixture at a pressure of at least 4000 psig (2.758 x 10⁷Pa), and
(iii) spray-drying the homogenized mixture,
the spray-dried active ingredient having an increased dissolution rate in water compared to the unencapsulated active ingredient.

2. A process according to claim 1, wherein the starch derivative is present in an amount of from 7 to 25% by weight, based on the total weight of the spray dried particle.

3. A process according to claim 1, wherein the amount of xanthan gum is from 0.3 to 0.6% by weight, based on the total weight of the mixture prior to spray-drying.

4. A process according to claim 1, wherein the active ingredient is present in an amount of from 3% to 25% by weight, based on the total weight of the mixture prior to spray-drying.

5. A process according to claim 1, wherein the homogenization step is performed in two or more passes through a homogenizer.

6. A process according to claim 1, wherein the starch derivative comprises an alkenyl-succinated starch, preferably an octenyl-succinated starch. Ideally the octenyl-succinated starch has a degree of substitution no greater than 0.03, more preferably no greater than 0.02.

7. A process according to claim 6, wherein the alkenyl-succinated starch is an octenyl-succinated starch.

8. A process according to claim 7, wherein the octenyl-succinated starch has a degree of substitution no greater than 0.03.

9. A process according to claim 1 wherein the homogenization step (ii) occurs in two complete passes.

10. A process according to claim 1 wherein the starch derivative is present in an amount of from 7 to 25% by weight, based on the total weight of the mixture prior to spray-drying.

11. A process according to claim 1 wherein the second carrier material is present in an amount of from 60 to 80% by weight, based on the total weight of the mixture prior to spray-drying.

12. A spray dried particle obtainable according to the process of claim 6.

## Patentansprüche

1. Verfahren zum Herstellen eines sprühgetrockneten Produkts zum Verbessern der Geschwindigkeit der Auflösung des Wirkstoffs, wobei das Verfahren die Schritte umfasst:
(i) Mischen eines kristallinen Wirkstoffs, welcher eine Auflösungsgeschwindigkeit in ungerührtem Wasser bei 25°C von mehr als 15 Minuten bei einer Konzentration von 14 ppm des Wirkstoffs hat, mit einem Stärkederivat, einem zweiten Trägermaterial, umfassend ein Maltodextrin mit einer mittleren Dextroseäquivalenz von 5 bis 25, und Xanthangummi, um eine Suspension, Dispersion oder Lösung des Wirkstoffs zu bilden,
(ii) Homogenisieren des Gemisches bei einem Druck von mindestens 4000 psig (2,758 x 10⁷ Pa) und
(iii) Sprühtrocknen des homogenisierten Gemisches,
wobei der sprühgetrocknete Wirkstoff eine erhöhte Auflösungsgeschwindigkeit in Wasser, verglichen mit dem uneingekapselten Wirkstoff, hat.

2. Verfahren gemäß Anspruch 1, wobei das Stärkederivat in einem Anteil von 7 bis 25 Gew.-%, bezogen auf das Gesamtgewicht der sprühgetrockneten Partikel, vorhanden ist.

3. Verfahren gemäß Anspruch 1, wobei der Anteil von Xanthangummi von 0,3 bis 0,6 Gew.-%, bezogen auf das Gesamtgewicht des Gemisches vor dem Sprühtrocknen, beträgt.

4. Verfahren gemäß Anspruch 1, wobei der Wirkstoff in einem Anteil von 3 Gew.-% bis 25 Gew.-%, bezogen auf das Gesamtgewicht des Gemisches vor dem Sprühtrocknen, vorhanden ist.

5. Verfahren gemäß Anspruch 1, wobei der Homogenisierungsschritt in zwei oder mehreren Durchgängen durch einen Homogenisierer vorgenommen wird.

6. Verfahren gemäß Anspruch 1, wobei das Stärkederivat eine Alkenylsuccinierte Stärke, vorzugsweise eine Octenyl-succinierte Stärke, umfasst, wobei die Octenyl-succinierte Stärke idealerweise einen Substitutionsgrad von nicht mehr als 0,03, stärker bevorzugt nicht mehr als 0,02 hat.

7. Verfahren gemäß Anspruch 6, wobei die Alkenyl-succinierte Stärke eine Octenyl-succinierte Stärke ist.

8. Verfahren gemäß Anspruch 7, wobei die Octenyl-succinierte Stärke einen Substitutionsgrad von nicht mehr als 0,03 hat.

9. Verfahren gemäß Anspruch 1, wobei der Homogenisierungsschritt (ii) in zwei vollständigen Durchgängen vonstatten geht.

10. Verfahren gemäß Anspruch 1, wobei das Stärkederivat in einem Anteil von 7 bis 25 Gew.-%, bezogen auf das Gesamtgewicht des Gemisches vor dem Sprühtrocknen, vorhanden ist.

11. Verfahren gemäß Anspruch 1, wobei das zweite Trägermaterial in einem Anteil von 60 bis 80 Gew.-%, bezogen auf das Gesamtgewicht des Gemisches vor dem Sprühtrocknen, vorhanden ist.

12. Sprühgetrocknete Partikel, erhältlich gemäß dem Verfahren nach Anspruch 6.

## Revendications

1. Procédé de préparation d'un produit séché par pulvérisation, pour améliorer la vitesse de dissolution de l'ingrédient actif, le procédé comprenant les étapes consistant à:
(i) mélanger un ingrédient actif cristallin, qui a une vitesse de dissolution dans de l'eau non agitée à 25°C supérieure à 15 minutes à une concentration de 14 ppm de l'ingrédient actif, avec un dérivé d'amidon, un second matériau support comprenant une maltodextrine ayant un Dextrose Equivalent moyen de 5 à 25, et de la gomme de xanthane pour former une suspension, dispersion ou solution de l'ingrédient actif,
(ii) homogénéiser le mélange à une pression d'au moins 4000 psig (2,758 x 10⁷ Pa), et
(iii) sécher par pulvérisation le mélange homogénéisé,
l'ingrédient actif séché par pulvérisation ayant une vitesse de dissolution accrue dans l'eau par rapport à l'ingrédient actif non encapsulé.

2. Procédé selon la revendication 1, dans lequel le dérivé d'amidon est présent en une quantité de 7 à 25% en poids, rapporté au poids total de la particule séchée par pulvérisation.

3. Procédé selon la revendication 1, dans lequel la quantité de gomme de xanthane est de 0,3 à 0,6% en poids, rapporté au poids total du mélange avant le séchage par pulvérisation.

4. Procédé selon la revendication 1, dans lequel l'ingrédient actif est présent en une quantité de 3 à 25% en poids, rapporté au poids total du mélange avant le séchage par pulvérisation.

5. Procédé selon la revendication 1, dans lequel l'étape d'homogénéisation est réalisée en deux passages ou plus à travers un homogénéiseur.

6. Procédé selon la revendication 1, dans lequel le dérivé d'amidon comprend un alcénylsuccinate d'amidon, de préférence un octénylsuccinate d'amidon, l'octénylsuccinate d'amidon ayant idéalement un degré de substitution non supérieur à 0,03, plus préférentiellement non supérieur à 0,02.

7. Procédé selon la revendication 6, dans lequel l'alcénylsuccinate d'amidon est un octénylsuccinate d'amidon.

8. Procédé selon la revendication 7, dans lequel l'octénylsuccinate d'amidon a un degré de substitution non supérieur à 0,03.

9. Procédé selon la revendication 1, dans lequel l'étape d'homogénéisation (ii) se fait en deux passages complets.

10. Procédé selon la revendication 1, dans lequel le dérivé d'amidon est présent en une quantité de 7 à 25% en poids, rapporté au poids total du mélange avant le séchage par pulvérisation.

11. Procédé selon la revendication 1, dans lequel le second matériau support est présent en une quantité de 60 à 80% en poids, rapporté au poids total du mélange avant le séchage par pulvérisation.

12. Particule séchée par pulvérisation pouvant être obtenue selon le procédé de la revendication 6.
